(19) Europäisches Patentamt
European Patent Office
Office européen des brevets

(11) Numéro de publication: **0 051 527 A1**

(12) # DEMANDE DE BREVET EUROPEEN

(21) Numéro de dépôt: 81401691.1

(22) Date de dépôt: 23.10.81

(51) Int. Cl.³: **C 07 C 43/13**
C 07 C 41/16, B 01 F 17/00

(30) Priorité: 24.10.80 FR 8022874

(43) Date de publication de la demande: 12.05.82 Bulletin 82/19

(84) Etats contractants désignés: BE IT

(71) Demandeur: Etablissement Public dit : CENTRE NATIONAL DE LA RECHERCHE SCIENTIFIQUE (CNRS)
15, Quai Anatole France
F-75700 Paris(FR)

(72) Inventeur: Leempoel, Patrick
125, Avenue de Messidor
B-1180 Bruxelles(BE)

(72) Inventeur: Selve, Claude
8, allée J.A. Baif
F-54600 Villers-Les-Nancy(FR)

(72) Inventeur: Mathis, Gérard
48 ter, avenue Anatole France
F-54000 Nancy(FR)

(72) Inventeur: Castro, Bertrand
89, rue Général Leclerc
F-54850 Messeix(FR)

(72) Inventeur: Delpuech, Jean-Jacques
16, rue du Plateau
F-54520 Laxou(FR)

(74) Mandataire: Phélip, Bruno et al,
c/o Cabinet Harlé & Phélip 21, rue de La Rochefoucauld
F-75009 Paris(FR)

(54) **Procédé pour la synthèse de composés hydrogénés et/ou fluorés du type polyoxyéthylène-alkyl-éther, composés de ce type et application à titre d'agents tensio-actifs non ioniques.**

(57) La présente invention concerne un procédé pour la synthèse de composés hydrogénés et/ou fluorés de type polyoxyéthylène-alkyl-éther.

Ce procédé consiste à faire réagir de la trisdiméthylaminophosphine sur un alcool de formule $H(OC_2H_4)_n$ OH en présence de tétrachlorure de carbone pour former le chlorure du monosel d'oxyphosphonium de formule:

$H(OC_2H_4)_n\ OP^+(NMe_2)_3,\ Cl^-$

en opérant dans un milieu dans lequel le glycol est soluble et dans lequel le monosel précipite et a une température suffisamment faible pour que le chlorure de monosel soit stable ; à substituer l'anion chlorure par un autre anion pour obtenir un monosel stable à une température supérieure à 0°C ; à effectuer une substitution nucléophile sur le sel d'oxyphosphonium du polyoxyéthylèneglycol ainsi formé par alcoolate de formule $R_2CH_2OM$.

Application: agents tensio-actifs non ioniques.

EP 0 051 527 A1

Procédé pour la synthèse de composés hydrogénés et/ou fluorés du type polyoxyéthylène-alkyl-éther, composés de ce type et application à titre d'agents tensio-actifs non ioniques.

La présente invention concerne un procédé pour la synthèse de composés hydrogénés et/ou fluorés du type polyoxyéthylène-alkyl-éther. Elle a également pour objet les composés de ce type et leur application à titre d'agents tensio-actifs non ioniques.

Les procédés utilisés jusqu'à présent pour la synthèse d'agents tensio-actifs fluorés non ioniques de formule générale $R_F(CH_2)_x(OC_2H_4)_n$ OH dans laquelle $R_F$ est une chaîne fluorée, sont basés sur la condensation de l'oxyde d'éthylène sur l'alcool fluoré de formule $R_F(CH_2)_xOH$.Le produit de la réaction de condensation est un mélange d'agents tensio-actifs présentant une répartition plus ou moins large du nombre n de motifs éther. Les produits purs, c'est-à-dire les composés fluorés ayant un nombre n de motifs éther bien défini, peuvent ensuite être obtenus par distillation. Cependant, les rendements ne sont que de l'ordre de 20 à 30 % environ [R. TATEMATSU et al. Yakagaku (1976), 25 p. 287 et Yakagaku (1980) 29, p. 23 ]. Cependant, la séparation par distillation ou chromatographie des composés fluorés ayant un nombre n élevé de motifs éther est très difficile à réaliser et il est donc pratiquement impossible d'obtenir un composé fluoré répondant à la formule ci-dessus, ayant un nombre de motifs n bien défini lorsque n est supérieur ou égal à 6.

On peut également citer, à titre de références concernant des procédés analogues d'obtention de ces agents tensio-actifs fluorés, les ouvrages ci-après :

-Z. chem. 1976,16(6) 229-30(l. Grenier et al)

- Yukagaku 1977, 26(6), 367-71 (R. Tatemasu et al)

Dans le brevet FR 73.39.533 publié sous le n° 2.249.657, il est indiqué que les tensio-actifs fluorés de type polyoxyéthylène-fluoroalkyl éther peuvent être facilement préparés par éthoxylation des alcools fluorés, mais que la séparation des différents produits d'éthoxylation avec des valeurs élevées de n (n étant le nombre de motifs oxyde d'éthylène) est difficile à réaliser et qu'en conséquence l'indice n correspond à une valeur moyenne (page 7, 1er paragraphe).

La demande de brevet JP 73.36.514, publiée sous le numéro 123.184/74 concerne l'obtention d'émulsions aqueuses contenant un dérivé fluorocarboné comme substituant principal, un agent tensioactif non ionique et de l'eau. Les agents tensio-actifs mis en oeuvre pour l'obtention de ces émulsions sont obtenus par addition d'oxyde d'éthylène à des alcools. Ils se présentent sous la forme de mélanges ayant une répartition conforme à une courbe de Gauss et non pas sous la forme de produits purs.

Les homologues hydrogénés de ces composés fluorés, c'est-à-dire les composés de formule $R_H(OC_2H_4)_n$ OH dans laquelle $R_H$ est une chaîne alkylée peuvent être obtenus par éthoxylation selon un procédé analogue à celui défini ci-dessus ou par addition de type Williamson d'un polyalcool sur un alcool ou par éthérification d'un tosilate. Ces procédés permettent d'obtenir un nombre de motifs éther bien défini avec des rendements de l'ordre de 20 à 50 %. A cet effet, on pourra se référer à l'article de SHICK "Non ionic surfactants" M. Dekker N.Y. 1966. Cependant, aucun de ces procédés d'addition de type Williamson ou d'éthérification d'un tosilate n'a été,

à la connaissance du demandeur, mis en oeuvre pour l'obtention d'agents tensio-actifs fluorés non ioniques.

Pour certaines applications, il est nécessaire d'obtenir des composés fluorés du type polyoxyéthylène-fluoro-alkyl-éther ayant un nombre bien défini de motifs éther. La présente invention permet l'obtention de tels composés fluorés avec en général des rendements d'au moins 30 %.

Le procédé selon l'invention pour la synthèse de composés hydrogénés et/ou fluorés de type polyoxyéthylène-alkyl-éther de formule générale :

$$R_Z-CH_2-(OC_2H_4)_n\ OH$$

dans laquelle :

$R_Z$ est une chaîne hydrocarbonée, fluorocarbonée ou fluorocarbonée partiellement hydrogénée et

<u>n</u> est un nombre entier au moins égal à 1,

consiste :

1) à faire réagir de la trisdiméthylaminophosphine sur un alcool de formule $H(OC_2H_4)_nOH$ en présence de tétrachlorure de carbone pour former le chlorure du monosel d'oxyphosphonium de formule :

$$H(OC_2H_4)_n\quad OP^+(NMe_2)_3,\ Cl^-$$

en opérant dans un milieu dans lequel le glycol est soluble et dans lequel le monosel précipite et à une température suffisamment faible pour que le chlorure du monosel soit stable ;

2) à substituer l'anion chlorure par un autre anion pour obtenir un monosel stable à une température supérieure à 0°C ;

3) à effectuer une substitution nucléophile sur le sel d'oxyphosphonium du polyoxyéthylèneglycol ainsi formé par un alcoolate de formule $R_ZCH_2OM$.

Par "chaîne fluorocarbonée" on désigne dans la présente description les chaînes alkyles linéaires

ou ramifiées complètement fluorées.

A titre d'exemples non limitatifs de chaînes fluorocarbonées partiellement hydrogénées on pourra citer notamment les chaînes fluorocarbonées hydrogénées en bout de chaîne.

La première étape du procédé de l'invention consiste en la formation du chlorure du monosel d'oxyphosphonium par réaction de la trisdiméthylaminophosphine sur un polyéthylèneglycol. Il importe d'opérer dans un milieu qui permet de solubiliser le glycol et dans lequel le monosel précipite immédiatement après sa formation. Le choix du milieu réactionnel dépend donc du glycol mis en oeuvre et du monosel formé. On indiquera à titre d'exemple que le tétrahydrofuranne convient comme milieu réactionnel pour les polyéthylèneglycols ayant 1 à 5 motifs éther. Pour un nombre de motifs éther supérieur à 5 on utilise avantageusement un mélange tétrahydrofuranne/éther. On opère avantageusement à une température comprise entre -20 et -40°C et sous atmosphère inerte, par exemple sous atmosphère d'azote. A une température supérieure, il se forme également du monosel, mais celui-ci n'est alors pas stable et se décompose immédiatement.

En général, on utilise avantageusement environ un équivalent molaire d'alcool de formule $H(OC_2H_4)_nOH$ pour environ un équivalent molaire de trisdiméthylaminophosphine (TDAP) en présence d'un excès de tétrachlorure de carbone, par exemple environ 2 à 5 équivalents molaires de tétrachlorure de carbone. Il est nécessaire d'ajouter lentement la trisdiméthylaminophosphine au mélange constitué de l'alcool, du tétrachlorure de carbone et du solvant. Après la fin de l'addition de TDAP on maintient avantageusement le milieu réactionnel à la même

température jusqu'à l'achèvement complet de la réaction, par exemple pendant 30 minutes environ. Le mélange réactionnel ainsi obtenu est ensuite jeté dans de l'eau et lavé à l'éther avant mélange avec une solution aqueuse de l'anion que l'on désire substituer. On utilise de préférence une solution aqueuse d'un excès d'hexafluorophosphate de potassium, contenant par exemple 2 équivalents molaires de ce sel. Le sel d'oxyphosphonium ainsi formé est ensuite extrait du mélange réactionnel à l'aide d'un solvant organique, par exemple le chlorure de méthylène et est ensuite séché.

On a déjà utilisé ce type de réaction pour la fabrication de sels d'alkyloxyphosphonium ou de sels d'alkyloxytrisdialkylaminophosphonium. A titre de références bibliographiques concernant ce type de réactions, on citera par exemple les articles ci-après :

-B.CASTRO et C.SELVE dans Bull. Soc. Chim. Fr 1974 p.3009 intitulé "sels d'alkyloxyphosphonium-Préparation de propanols bisubstitués en 2 et fonctionnalisés en 3" ;

-R BOIGEGRAIN et al. dans Tetrahedron Letters n° 30 p. 2529-2530, 1975 intitulé "sels d'alkyloxyphosphonium monoactivation sélective des diols-1, n biprimaires symétriques dans les réactions de substitutions nucléophiles" ;

-le brevet FR 73.17.883 concerne aussi l'obtention de sels d'alkyloxytrisdialkylaminophosphonium fonctionnels.

La troisième étape du procédé de l'invention consiste en une substitution nucléophile sur le sel d'oxyphosphonium de polyoxyéthylèneglycol ainsi obtenu par un alcoolate fluoré de formule $R_Z(CH_2)OM$, dans laquelle M est un cation, par exemple

le sodium. L'alcoolate fluoré en solution dans un solvant organique aprotique est mis à réagir par chauffage avec le sel d'oxyphosphonium de polyoxyéthylèneglycol. On opère avantageusement à une température comprise entre 50 et 100°C, par exemple à 60°C pendant 8 et 48 heures, notamment pendant 24 heures, sous atmosphère inerte et agitation. Les solvants de type éther, mono- ou poly-éther à point d'ébullition élevé sont particulièrement appropriés, tels que par exemple le diméthoxyéthane, le dioxane, ce dernier étant particulièrement préféré. On peut également utiliser les mélanges de tels solvants. On utilise en général environ 1 équivalent molaire d'alcoolate fluoré pour 1 équivalent molaire de sel d'oxyphosphonium de polyoxyéthylèneglycol. Le mélange réactionnel ainsi obtenu est évaporé et le résidu est extrait avec par exemple de l'éther. La phase organique résultante est ensuite lavée et séchée, puis les produits formés sont préparés par chromatographie. L'éluant chromatographique peut être par exemple l'acétate d'éthyle ou un mélange acétate d'éthyle/méthanol.

Le procédé selon l'invention peut être représenté par le schéma réactionnel ci-après :

$$H(OC_2H_4)_nOH + P(N\ Me_2)_3 + CCl_4 \xrightarrow[2)\ KPF_6]{1)\ solvant}$$

$$H(OC_2H_4)_n\ OP^+\ (N\ Me_2)_3\ \ PF_6^- + CHCl_3 + KCl$$

$$R_Z\ CH_2\ OM\ +\ H(OC_2H_4)_n\ \ OP^+(N\ Me_2)_3\ PF_6^- \xrightarrow{solvant}$$

$$R_Z\ CH_2\ (OC_2H_4)_n\ \ OH\ + HMPT + MPF_6$$

Les composés de départ mis en oeuvre dans le procédé de l'invention, à savoir le polyoxyéthylèneglycol de formule $H(OC_2H_4)_n$ OH et l'alcoolate

fluoré $R_Z$ $CH_2$ OM sont obtenus selon des moyens classiques. Par exemple, le polyoxyéthylèneglycol $H(OC_2H_4)_n$ OH est obtenu par condensation d'oxyde d'éthylène avec un glycol ou par un procédé analogue /"Non Ionic Surfactants" ed. M SHICK M. DEKKER 1966 _7.

L'alcoolate fluoré peut être obtenu par réaction d'un méthylate, par exemple du méthylate de sodium en solution méthanolique avec l'alcool fluoré de formule $R_Z$ $CH_2$ OH et évaporation sous vide du solvant, par exemple le méthanol.

La présente invention concerne également les composés obtenus par le procédé ci-dessus. Elle concerne ainsi, à titre de produits nouveaux, les composés fluorés de formule $R_FCH_2$ $(OC_2H_4)_n$ OH dans laquelle n est supérieur ou égal à 6 et $R_F$ est une chaîne fluorocarbonée ou fluorocarbonée partiellement hydrogénée.

Les composés selon l'invention possèdent les propriétés caractéristiques des agents tensio-actifs non ioniques et conviennent en conséquence comme agents tensio-actifs non ioniques. La présente invention a donc également pour objet l'application des composés fluorés définis ci-dessus comme agents tensio-actifs.

Grâce à ces propriétés tensio-actives non ioniques les composés selon l'invention peuvent être utilisés notamment comme agents émulsionnants.

L'invention va être maintenant décrite plus en détail par les exemples illustratifs non limitatifs ci-après :

EXEMPLE 1

A. Préparation du monosel d'oxyphosphonium de tétra oxyéthylèneglycol.

Dans un réacteur muni d'un agitateur magnétique et d'une ampoule à brome, on a introduit un équivalent (9,71 g) de tétraoxyéthylèneglycol et 2,5 équivalents (19,23 g) de tétrachlorure de carbone dans 25 à 50 ml de solvant anhydre (tétrahydrofuranne), l'ampoule contenant une solution d'un équivalent (8,16 g) de trisdiméthylaminophosphine (TDAP).

L'ensemble du montage réactionnel a été placé sous atmosphère d'azote avant d'être refroidi à environ -40°C.

On a additionné alors lentement la TDAP. L'addition a duré 3 heures et une fois l'addition terminée on a laissé réagir 30 minutes au plus.

Le mélange réactionnel a alors été jeté sur 100 ml d'eau et le réacteur a été lavé ultérieurement deux fois.

La phase aqueuse a été lavée à l'éther avant mélange avec une solution aqueuse de 2 équivalents (18,4 g) d'hexafluorophosphate de potassium.

L'extraction du sel d'oxyphosphinium a été effectuée ensuite par lavage au chlorure de méthylène. Cette phase organique a été séchée sur $Na_2SO_4$ avant évaporation au rotavapor.

On a obtenu 23,3 g du monosel $H(OC_2H_4)_4OP^+(N\ Me_2)_3\ PF_6^-$ sous la forme d'un liquide visqueux.

<u>B - Synthèse du composé fluoré de formule</u>

$C_7F_{15}CH_2(OC_2H_4)_4OH$

On a tout d'abord préparé l'alcoolate fluoré de formule $C_7F_{15}CH_2O\ Na$ ; 0,25 g de sodium a été ajouté à 50 ml de méthanol. A cette solution méthanolique de 1,1 équivalent de méthylate de sodium , on a ajouté 1,2 équivalent (4,8 g) de l'alcool fluoré $C_7F_{15}CH_2OH$. L'évaporation sous vide du solvant a permis l'obtention du fluoroalcoolate, que l'on a solubilisé ultérieurement dans 50 ml de

dioxane en présence d'un équivalent du sel d'oxyphosphonium du tétraoxyéthylèneglycol obtenu selon le mode opératoire ci-dessus.

La solution ainsi obtenue a été chauffée 24 heures à 60°C sous atmosphère d'azote et agitation magnétique.

Après réaction le mélange réactionnel a été évaporé et le résidu a été extrait à l'éther. Cette phase organique a été lavée avec une solution acide HCl 2N (3 x 100 ml) et séchée sur $MgSO_4$. Les produits de réaction ont été séparés chromatographiquement en utilisant de l'acétate d'éthyle comme éluant. La chromatographie a été réalisée sur silice (120 g).

On a ainsi obtenu 2,3 g d'un liquide jaunâtre dont l'analyse élémentaire était la suivante :

| | | C | F | H |
|---|---|---|---|---|
| $C_7F_{15}CH_2(OC_2H_4)_4OH$ | trouvé | 33,15 | 49,08 | 3,38 |
| | calculé | 33,34 | 49,45 | 3,32 |

De plus, ce composé présentait les caractéristiques physico-chimiques ci-après :

- point de trouble < 0°C
- indice hydrophile-liphophile HLB 3,65
- $n_D^{21}$ 1,375
- rendement : 40 % par rapport au glycol mis en oeuvre.

Spectre RMN $^1H$ $R_FCH_2(OC_2H_4)_nOH$, $R_F = C_7F_{15}$
a b c

solvant $CDCl_3$ $\delta_{ppm}$/TMS
a : triplet détriplé 4,08 ppm
b : massif 3,69 ppm
c : 3,24

Constance de couplage $J^4_{HF_a}$ : 14,1 Hz $J^5_{HF_a}$ =1,6Hz

EXEMPLE 2

Synthèse du composé fluoré de formule

$C_7F_{15}CH_2(OC_2H_4)_5OH$

On a opéré selon le même mode opératoire qu'à l'exemple 1 (A) pour former le monosel d'oxyphosphonium de pentaoxyéthylène glycol en utilisant les ingrédients ci-après :

| | |
|---|---|
| Pentaoxyéthylèneglycol | 11,9 g |
| TDAP | 8,15 g |
| $CCl_4$ | 19,25 g |
| $KPF_6$ | 18,5 g |

La température d'addition du TDAP était de -35°C ; celle-ci a été effectuée pendant 3 heures et le solvant utilisé était le tétrahydrofuranne. On a obtenu 25 g du monosel $H(OC_2H_4)_5\ OP^+(N(Me)_2)_3\ PF_6^-$ (liquide visqueux).

On a ensuite fait réagir 5,45 g de ce monosel d'oxyphosphonium avec l'alcoolate fluoré de formule $C_7F_{15}CH_2O$ Na, obtenu en faisant réagir 0,25 g de sodium avec 50 ml de méthanol, puis avec 4,75 g du composé de formule $C_7F_{15}CH_2OH$ selon le mode opératoire défini à l'exemple 1(B). La solution de l'alcoolate fluoré $C_7F_{15}CH_2ONa$ dans le dioxane 50 ml avec le monosel d'oxyphosphonium de pentaoxyéthylèneglycol a été chauffée pendant 24 heures à 60°C. Le résidu extrait à l'éther a été lavé et séché sur $MgSO_4$. Après chromatographie sur silice (120 g ; éluant : acétate d'éthyle/méthanol 95/5), on a obtenu 2,4 g d'un liquide jaunâtre qui présentait les caractéristiques physico-chimiques ci-après:

- point de trouble $<$ 0°C
- indice hydrophile-lipophile HLB : 3,98
- $n_D^{21} = 1,382$
- rendement/glycol : 38,7 %

<u>EXEMPLE 3</u>

<u>Synthèse du composé fluoré de formule</u>

$C_7F_{15}CH_2(OC_2H_4)_6OH$

On a opéré sensiblement selon le même mode opératoire que celui décrit à l'exemple 1 en utilisant les ingrédients ci-après :

0,26 g de sodium dans 50 ml de méthanol

4,82 g de $C_7F_{15}CH_2OH$

50 ml de dioxane

5,89 g de monosel d'oxyphosphonium de l'hexaoxyéthylèneglycol.

Le monosel de l'hexaoxyéthylèneglycol a été obtenu en faisant réagir les composés ci-après selon le mode opératoire de l'exemple 1 (A) :

| | |
|---|---|
| Hexaoxyéthylèneglycol | 2,82 g |
| TDAP | 1,63 g |
| $CCl_4$ | 3,85 g |
| $KPF_6$ | 3,68 g |

dans les conditions ci-après :

Température d'addition -28°C solvant tétrahydrofuranne/éther 3/1

Temps d'addition : 3 H.

On a obtenu 5,40 g du monosel de l'hexaoxyéthylèneglycol sous la forme d'un liquide visqueux.

La réaction de substitution nucléophile a été réalisée pendant 24 heures à 62°C. Après extraction, lavage, séchage et chromatographie sur silice (120 g ; éluant ; acétate d'éthyle/méthanol 95/5), on a obtenu 2,6 g d'un liquide jaunâtre dont l'analyse élémentaire était la suivante :

| | | C | F | H |
|---|---|---|---|---|
| $C_7F_{15}CH_2(OC_2H_4)_6OH$ | trouvé | 36,13 | 43,55 | 4,13 |
| | calculé | 36,15 | 42,89 | 4,10 |

point de trouble $<$ 0°C

HLB 4,31

$n_D^{21}$ 1,389

rendement/glycol 39,1 %

Spectre RMN

Solvant $CCl_4$ $\delta_{ppm}$/TMS a = 4,09 ppm b = 3,59 ppm c = 3,70

Constante de couplage $J^4_{HF_a}$ = 14,0 Hz $J^5_{HF_a}$ = 1,6 Hz

EXEMPLE 4

Synthèse du composé fluoré de formule

$C_6F_{13}CH_2(OC_2H_4)_5OH$

On a opéré sensiblement selon le même mode opératoire que celui décrit à l'exemple 1 en utilisant les ingrédients ci-après :

0,25 g de sodium dans 50 ml de méthanol

4,2 g de $C_6F_{13}CH_2OH$

50 ml de dioxane

5,4 g du monosel d'oxyphosphonium de pentaoxyéthylèneglycol.

La réaction de substitution nucléophile a été réalisée pendant 24 heures à 60°C ; après traitement du résidu et chromatographie (120 g de silice ; éluant : acétate d'éthyle/méthanol 95/5) on a obtenu 2,20 g d'un liquide jaunâtre dont l'analyse élémentaire a permis d'identifier le composé de formule $C_6F_{13}CH_2(OC_2H_5)_5OH$.

Ce composé présentait les caractéristiques physico-chimiques ci-après :

point de trouble ~ 0°C

HLB : 4,85

$n_D^{21}$ : 1,385

Spectre RMN

Solvant $CDCl_3$ $\delta_{ppm}$/TMS a = 4,11 ppm

b = 3,66 ppm

Rendement/glycol : 39 % $J^4_{HF_a}$ = 14 Hz

EXEMPLE 5

Synthèse du composé fluoré de formule

$C_6F_{13}CH_2(OC_2H_4)_6OH$

On a opéré sensiblement selon le même mode opératoire que celui décrit à l'exemple 1 en utilisant les ingrédients ci-après :

0,25 g de sodium

50 ml de méthanol

4,2 g $C_6F_{13}CH_2OH$

50 ml de dioxane

5,9 g du monosel d'hexaoxyéthylèneglycol.

On a chauffé le milieu réactionnel à 60°C pendant 24 heures. Après traitement du résidu et chromatographie (120 g silice ; éluant : acétate d'éthyle/méthanol 95/5), on a obtenu 2,35 g d'un liquide jaunâtre présentant les caractéristiques ci-après :

point de trouble en solution aqueuse à 1 % = 10,5°C

HLB = 5,18

$n_D^{21}$ = 1,393

Spectre RMN dans $CDCl_3$ $\delta_{ppm}$/TMS a = 4,14ppm

b = 3,63ppm

$J^4_{HF_a}$=14,0 Hz

Rendement/glycol 38,2 %

| Analyse élémentaire | C | H |
|---|---|---|
| trouvé | 36,85 | 4,52 |
| calculé | 37,14 | 4,43 |

## REVENDICATIONS

1. Procédé pour la synthèse de composés hydrogénés et/ou fluorés du type polyoxyéthylène-alkyl-éther de formule générale :

$$R_Z - CH_2 - (OC_2H_4)_n\ OH$$

dans laquelle :

$R_Z$ est une chaîne hydrocarbonée, fluorocarbonée ou fluorocarbonée partiellement hydrogénée

n est un nombre entier au moins égal à 1, consistant

1) à faire réagir de la trisdiméthylaminophosphine sur un alcool de formule $H(OC_2H_4)_n\ OH$ en présence de tétrachlorure de carbone pour former le chlorure du monosel d'oxyphosphonium de formule :

$$H\ (OC_2H_4)_n\ \ OP^{+}(NMe_2)_3\ \ Cl^{-}$$

en opérant dans un milieu dans lequel le glycol est soluble et dans lequel le monosel précipite et à une température suffisamment faible pour que le chlorure du monosel soit stable ;

2) à substituer l'anion chlorure par un autre anion pour obtenir un monosel stable à une température supérieure à 0°C ;

3) à effectuer une substitution nucléophile sur le sel d'oxyphosphonium du polyoxyéthylèneglycol ainsi formé par un alcoolate de formule $R_ZCH_2OM$.

2. Procédé selon la revendication 1, caractérisé en ce que la première étape est réalisée dans un solvant ou un mélange de solvants organiques anhydres à une température comprise entre - 20 et - 40°C.

3. Procédé selon l'une des revendications 1 ou 2, caractérisé en ce que l'on utilise du tétrahydrofuranne ou mélange tétrahydrofuranne/éther comme solvant organique.

4. Procédé selon l'une quelconque des revendications 1 à 3, caractérisé en ce que l'on utilise l'anion hexafluorophosphate dans la seconde étape.

5. Procédé selon l'une quelconque des revendications 1 à 4, caractérisé en ce que la substitution nucléophile sur le sel d'oxyphosphonium de polyoxyéthylèneglycol par l'alcoolate de formule $R_Z(CH_2)OM$ est réalisée par mélange dudit alcoolate en solution dans un solvant organique aprotique avec le sel d'oxyphosphonium de polyoxyéthylèneglycol, puis chauffage pendant 8 à 48 heures à une température comprise entre 50 et 100°C.

6. Procédé selon la revendication 5, caractérisé en ce que l'alcoolate est un alcoolate fluoré et en ce qu'il est utilisé en solution dans un éther à point d'ébullition élevé, notamment le dioxane.

7. Procédé selon l'une quelconque des revendications 1 à 6, caractérisé en ce que l'on utilise au cours de l'étape (1) environ un équivalent molaire d'alcool de formule $H(OC_2H_4)_n$ OH pour environ un équivalent molaire de trisdiméthylaminophosphine en présence d'environ 2 à 5 équivalents molaire de tétrachlorure de carbone et en ce qu'on utilise au cours de l'étape (3) environ 1 équivalent molaire d'alcoolate fluoré pour environ 1 équivalent molaire de sel d'oxyphosphonium de polyoxyéthylèneglycol.

8. A titre de produits nouveaux les composés fluorés de type polyoxyéthylène-fluoro-alkyl-éther de formule générale :

$$R_F(CH_2)(OC_2H_4)_n\ OH$$

dans laquelle

$R_F$ est une chaîne alkyl-fluorée

<u>n</u> est un nombre entier au moins égal à 1.

9. Composés fluorés selon la revendication 6,

caractérisés en ce que n est supérieur ou égal à 6.

10. Application des composés selon l'une quelconque des revendications 6 à 8, à titre d'agents tensio-actifs non ioniques.

Office européen des brevets

**RAPPORT DE RECHERCHE EUROPEENNE**

Numéro de la demande
EP 81 40 1691

## DOCUMENTS CONSIDERES COMME PERTINENTS

| Catégorie | Citation du document avec indication, en cas de besoin, des parties pertinentes | Revendication concernée |
|---|---|---|
| DX | FR - A - 2 249 657 (U. KUHLMANN)<br>* Pages 6,7 *<br>-- | 8,9, 10 |
| DX | CHEMICAL ABSTRACTS, vol. 83, no. 2, 14 juillet 1975, page 349, réf. 15661d<br>Columbus, Ohio, US<br>JP - A - 74 123 184 (ASAHI DENKA KOGYO K.K.) (25-11-1974)<br>* En entier *<br>-- | 8,9,10 |
| XD | CHEMICAL ABSTRACTS, vol. 85, 1976 abrégé 93732h, page 584<br>Columbus, Ohio, US<br>R. TATEMATSU et al.: "Preparation and properties of some fluoro-alcoholethylene oxide adducts"<br>& YUKAGAKU, 1976, 25 (5), 287-91<br>* En entier *<br>-- | 8,9, 10 |
| XD | CHEMICAL ABSTRACTS, vol. 85, 1976 abrégé 80056r, page 128<br>Columbus, Ohio, US<br>A. GREINER et al.: "Fluorinated surfactants based on telomeric alcohols; 1. Ethylene oxide adducts"<br>& Z. Chem. 1976, 16 (6), 229-30<br>* En entier *<br>-- | 8,9, 10 |
| XD | CHEMICAL ABSTRACTS, vol. 87, 1977 abrégé 103564n, page 65<br>Columbus, Ohio, US<br>./. | 8,10 |

**CLASSEMENT DE LA DEMANDE (Int. Cl.³)**

C 07 C 43/13
41/16
B 01 F 17/00

**DOMAINES TECHNIQUES RECHERCHES (Int. Cl.³)**

C 07 C 43/13
41/16

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 16-12-1981 | VERHULST |

Office européen des brevets

**RAPPORT DE RECHERCHE EUROPEENNE**

Numéro de la demande
EP 81 40 1691



| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| | R. TATEMATSU et al.: "Preparation and properties of some fluoro-alcohol-ethylene oxide adducts"<br>* En entier *<br>-- | | |
| D | TETRAHEDRON LETTERS, no. 30, juillet 1975, pages 2529-2530 Pergamon Press: Oxford, GB<br>R. BOIGEGRAIN et al.: "Sels d'alkyloxyphosphonium XIII(1). Monoactivation selective des diols-1,n biprimaires symetriques dans les reactions de substitutions nucleophiles"<br>* En entier *<br>-- | 1 | DOMAINES TECHNIQUES RECHERCHES (Int. Cl. 3) |
| D | BULLETIN DE LA SOCIETE CHIMIQUE DE FRANCE, décembre 1974, deuxième partie, Masson et Cie dépositaires 120, bd. St-Germain, Paris, FR, page 3009<br>B. CASTRO et al.: "Sels d'alkyl-oxyphosphonium. XI.-Préparation de propanols bisubstitués en 2 et fonctionnalisés en 3."<br>* Page 3009 *<br>---- | 1 | |